# EUROPEAN PATENT SPECIFICATION

(11) **EP 1 970 011 B1**
(45) Date of publication and mention of the grant of the patent: **15.06.2011**
(21) Application number: 08004765.7
(22) Date of filing: 14.03.2008
(51) Int. Cl.: A61B 8/06

(54) **Ultrasound diagnostic system and method for displaying a doppler spectrum image**
Ultraschalldiagnosesystem und Verfahren zur Anzeige eines Doppler-Spektrumbildes
Système de diagnostic à ultrasons et procédé d'affichage d'une image de spectre Doppler

(30) Priority: 16.03.2007 KR 20070026173
(43) Date of publication of application: 17.09.2008
(73) Proprietor: MEDISON CO., LTD., Kangwon-do 250-870 (KR)
(72) Inventor: Kim, Jong Sik, Seoul 135-280 (KR); Kim, Chan Mo, Seoul 135-280 (KR)
(74) Representative: Schmid, Wolfgang

(56) References cited:
- EP-A- 1 016 880
- EP-A- 1 769 747
- WO-A-2006/043603
- US-A1- 2006 084 873

## Description

The present application claims priority from Korean Patent Application No. 10-2007-0026173 filed on March 16, 2007, the entire subject matter of which is incorporated herein by reference.

### BACKGROUND OF THE INVENTION

### [Technical Field]

The present invention generally relates to ultrasound diagnostic systems, and more particularly to an ultrasound diagnostic system and a method for displaying a Doppler spectrum image.

### [Background Art]

The ultrasound diagnostic system has become an important and popular diagnostic tool due to its non-invasive and non-destructive nature. Modem high-performance ultrasound imaging diagnostic systems and techniques are commonly used to produce two- or three-dimensional images of internal features of patients.

An ultrasound diagnostic system generally uses a probe containing an array of piezoelectric elements to transmit and receive ultrasound signals. The ultrasound diagnostic system forms an image of human internal tissues by electrically exciting transducer elements to generate ultrasound signals that travel into the body. Echoes reflected from tissues and organs return to the transducer element and are converted into electrical signals, which are amplified and processed to produce a diagnostic image.

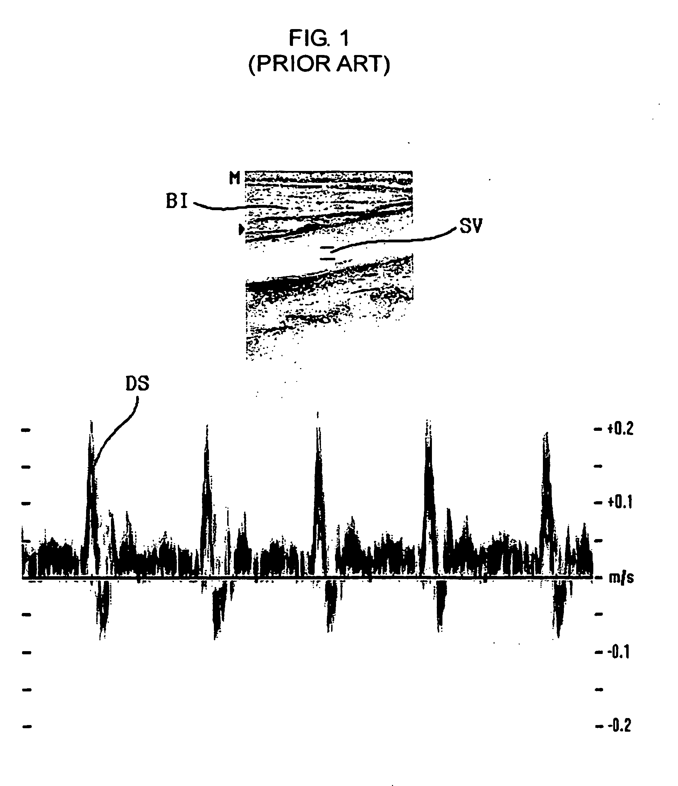

In the ultrasound diagnostic system, the Doppler effect is used to measure the velocity of red blood cells flowing within a blood vessel or the velocity of heart motion. FIG. 1 shows an example of displaying a B-mode image and a Doppler spectrum image at the same time. The B-mode image BI is an image that displays the brightness, which indicates the intensities of the ultrasound signals reflected from the target object, on a screen. If a user sets a sample volume SV on a blood vessel in the B-mode image BI by using a user input interface such as a track ball, then the ultrasound diagnostic system repeatedly transmits/receives ultrasound signals to/from a region corresponding to the sample volume. The ultrasound diagnostic system computes spectral Doppler components based on the reception signals and provides a Doppler spectrum image DS or sound corresponding to the frequency or velocity based on the computed spectral Doppler components. The Doppler spectrum image DS may indicate the motion direction and motion velocity of a moving object such as red blood cells or heart. In the Doppler spectrum image DS, a horizontal axis represents the time, while a vertical axis represents the velocity (or frequency).

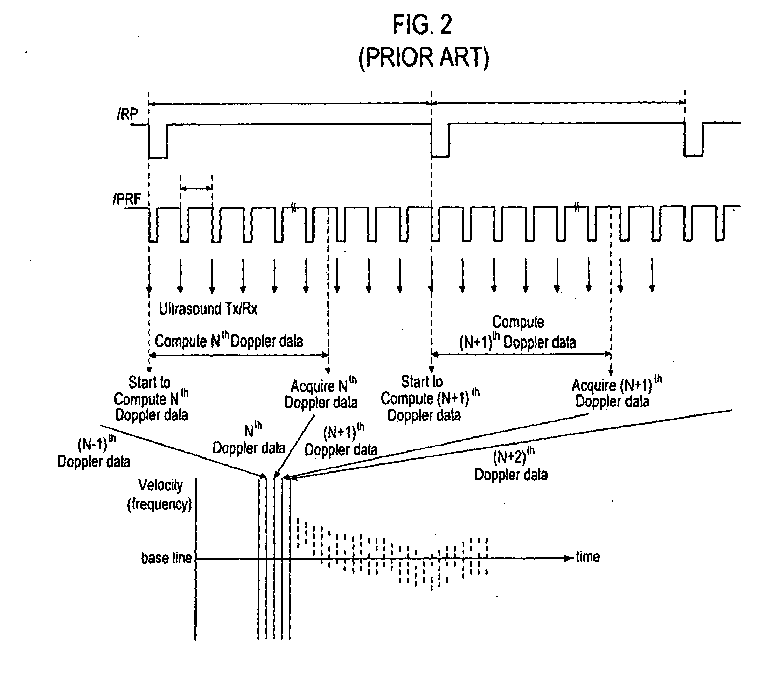

FIG. 2 is a schematic diagram showing an example of computing the spectral Doppler components. Referring to FIG. 2, a sample volume is set on the B-mode image. The ultrasound diagnostic system transmits/receives ultrasound signals to/from a region corresponding to the sample volume at a pulse repetition frequency (PRF). The ultrasound diagnostic system acquires ultrasound data based on the received ultrasound echo signals. The ultrasound diagnostic system computes the spectral Doppler components from the ultrasound data at a repetition period (RP) to obtain Doppler spectrum data. The RP is usually determined according to a sweep speed, which is adjustable by a user through an input unit such as a keyboard, a trackball or the like, in the conventional ultrasound diagnostic system. The sweep speed represents the time for scanning the sample volume. The time interval for displaying each Doppler spectrum in the Doppler spectrum image depends on the sweep speed.

If the user selects the sweep speed, then the ultrasound diagnostic system sets RP corresponding to the selected sweep speed to compute spectral Doppler components and obtains Doppler spectrum data based on the computed spectral Doppler components. The obtained Doppler spectrum data may be stored in a memory such as a buffer. In displaying the Doppler spectrum image based on the stored Doppler spectrum data, the sweep speed may be adjusted to be slower or faster so as to magnify or de-magnify the Doppler spectrum image on a time axis. In such a case, there is a problem in that the resolution of the Doppler spectrum image is limited. Also, since the RP for computing the Doppler components is determined according to the sweep speed, an adjustment of the sweep speed is limited (especially the maximum sweep speed).

WO 2006/043603 A describes an ultrasonic Doppler diagnosis device for receiving a reflected wave of an ultrasonic continuous wave in parallel to the transmission of the ultrasonic continuous wave in the range direction. The device includes a modulation unit for subjecting the ultrasonic continuous wave to frequency modulation so as to change the phase in accordance with the distance in the range direction, a demodulation unit for demodulating the reflected wave for each range in the range direction interlocked with the frequency modulation, and a presentation unit for presenting information by using the signal of the Doppler component based on the reception signal.

### BRIEF DESCRIPTION OF THE DRAWINGS

FIG. 1 is a photo showing an example of simultaneously displaying a B-mode image and a Doppler spectrum image.

FIG. 2 is a schematic diagram showing a procedure of displaying a Doppler spectrum image according to the prior art.

FIG. 3 is a block diagram showing an ultrasound diagnostic system constructed in accordance with the present invention.

FIG. 4 is a schematic diagram showing a procedure of displaying a Doppler spectrum image in accordance with the present invention.

FIG. 5 is a flowchart showing a method of displaying a Doppler spectrum image in accordance with the present invention.

### DETAILED DESCRIPTION OF THE INVENTION

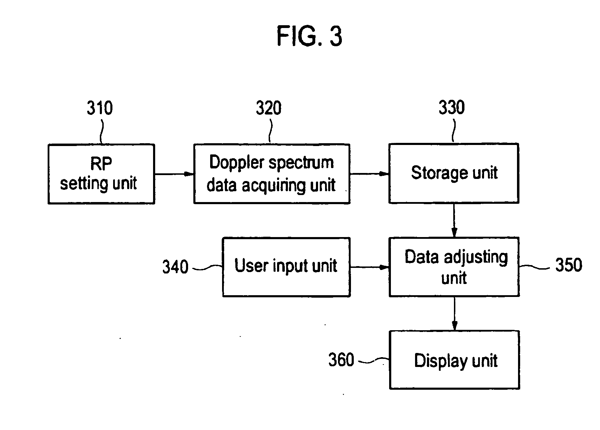

FIG. 3 is a block diagram showing an ultrasound diagnostic system constructed in accordance with the present invention. Referring to FIG. 3, the ultrasound diagnostic system 300 include a repetition period (RP) setting unit 310, a Doppler spectrum data acquiring unit 320, a storage unit 330, a user input unit 340, a data adjusting unit 350 and a display unit 360.

The RP setting unit 310 may be operable to calculate the fastest computable time necessary for computing spectral Doppler components from ultrasound data, which are obtained by transmitting/receiving ultrasound signals at a pulse repetition frequency (PRF), in the ultrasound diagnostic system. The RP setting unit 310 may add a predetermined margin to the calculated time to thereby set an RP. The RP may depend on the performance of the ultrasound diagnostic system. The predetermined margin, which is determined by considering a delay occurring in the ultrasound diagnostic system, may be set to about 1-10 % of the calculated time for a stable operation of the ultrasound diagnostic system.

The Doppler spectrum data acquiring unit 320 may be operable to compute the spectral Doppler components from the ultrasound data at the RP to thereby acquire Doppler spectrum data constituted with spectral Doppler components. The acquired Doppler spectrum data may be stored in the storage unit 330 such as a buffer, etc.

The user input unit 340 may enable a user to select the sweep speed. The sweep speed may affect the time interval at which a spectral Doppler component such as velocity or frequency is computed and displayed on a screen of the display unit 360, wherein the time interval is referred to as a Doppler display interval. The data adjusting unit 350 may be operable to read out the Doppler spectrum data stored in the storage unit 330 and compare the sweep speed with the RP to properly adjust the Doppler spectrum data. For example, if the sweep speed is faster than the RP, then the data adjusting unit 350 may be operable to interpolate the Doppler spectrum data such that the Doppler display interval is identical to the selected sweep speed. On the other hand, if the sweep speed is slower than the RP, then the data adjusting unit 350 may be operable to decimate the Doppler spectrum data such that the Doppler display interval is identical to the sweep speed. The display unit 360 may display the Doppler spectrum image based on the adjusted Doppler spectrum data.

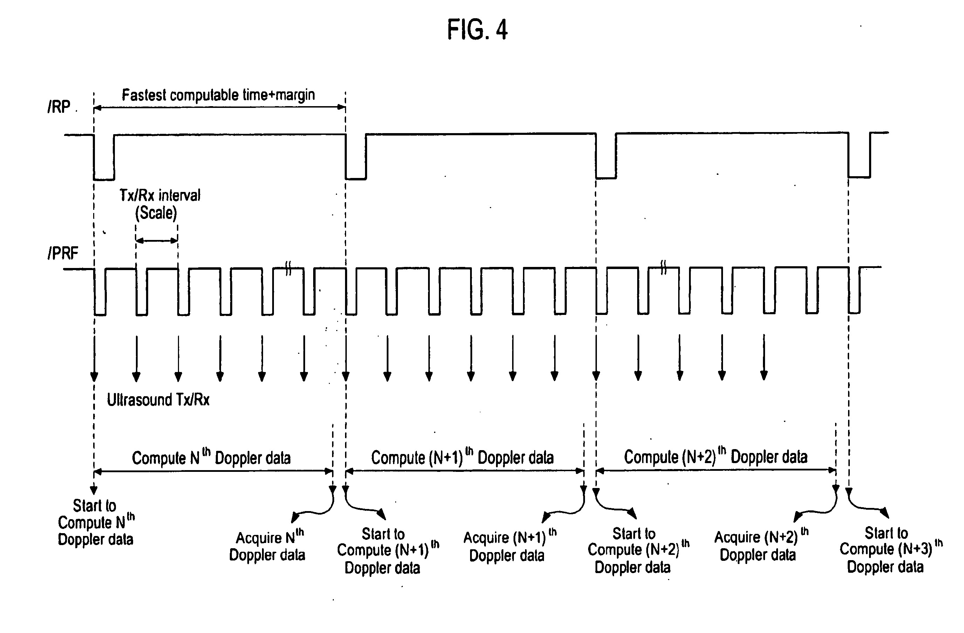

FIG. 4 is a schematic diagram showing a procedure of displaying a Doppler spectrum image in accordance with the present invention. As illustrated in FIG. 4, the RP setting unit 310 may be operable to calculate the fastest computable time and add the predetermined margin to the calculated time to thereby set the RP. That is, the RP may be determined regardless of the sweep speed in accordance with one embodiment of the present invention.

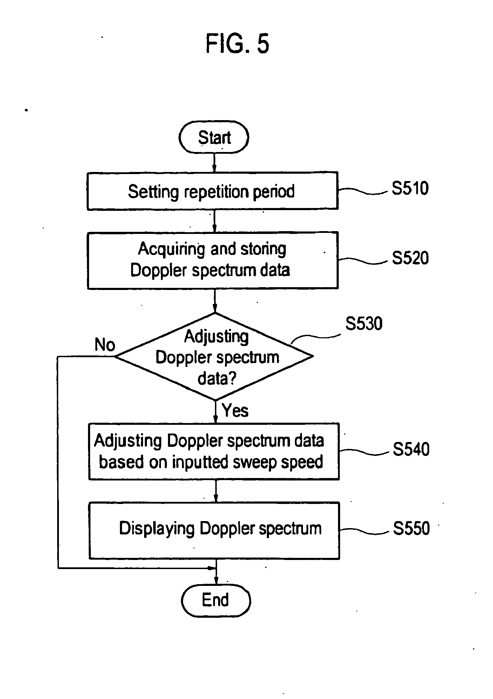

Hereinafter, a method of displaying the Doppler spectrum image in accordance with the present invention will be described. FIG. 5 is a flowchart showing a method of displaying a Doppler spectrum image.

Referring to FIG. 5, the fastest computable time for computing spectral Doppler components from ultrasound data obtained by transmitting/receiving ultrasound signals to/from the target object may be calculated and a predetermined margin is added to the calculated fastest computable time to thereby set a RP at step S510. The ultrasound diagnostic system transmits the ultrasound signals at a pulse repetition frequency (PRF) to a predetermined region in the target object to obtain the ultrasound data. The spectral Doppler components may be computed from the ultrasound data at the set RP to thereby obtain the Doppler spectrum data at step S520. The Doppler spectrum data may be stored in the storage unit 330.

Thereafter, if the sweep speed information for selecting a sweep speed is inputted from the user, then the sweep speed is compared with the RP to determine the adjustment of the Doppler spectrum data at step S530. If the sweep speed does not coincide with the RP, then the Doppler spectrum data may be adjusted through an interpolation process or a decimation process at step S540. That is, if the sweep speed is faster than the RP, then the interpolation process may be carried out upon the Doppler spectrum data. However, if the sweep speed is slower than the RP, then the decimation process may be carried out upon the Doppler spectrum data. For example, when the Doppler spectrum data are acquired at the RP of 1 KHz and the sweep speed inputted from the user is 100Hz, 200Hz or 500Hz, the Doppler spectrum data may be decimated at a ratio of 10:1, 5:1 or 2:1. On the other hand, when the inputted sweep speed is 2 KHz, the Doppler spectrum data may be interpolated at a ratio of 1:2. The Doppler spectrum image may be displayed based on the adjusted Doppler spectrum data.

As mentioned above, since the acquisition period of the Doppler spectrum data is fixed to a maximum computable period, the period for displaying the Doppler spectrum image may be easily adjusted without incurring any degradation of the Doppler spectrum image.

In accordance with one embodiment of the present invention, there is provided an ultrasound diagnostic system configured to display a Doppler spectrum image, comprising: a period setting unit operable to set a repetition period; a Doppler spectrum data acquiring unit operable to compute spectral Doppler components at the set repetition period from the ultrasound data representative of a target object, said computed spectral Doppler components being used to acquire Doppler spectrum data; a storage unit operable to store the acquired Doppler spectrum data; a user input unit operable to enable to a user to select a sweep speed; a data adjusting unit operable to compare the set repetition period with the selected sweep speed to adjust the Doppler spectrum data; and a display unit operable to display a Doppler spectrum image based on the adjusted Doppler spectrum data.

In accordance with another embodiment of the present invention, there is provided a method of displaying a Doppler spectrum image in an ultrasound diagnostic system, comprising: setting a repetition period; computing the spectral Doppler components at the set period from ultrasound data representative of a target object; acquiring Doppler spectrum data based on the computed spectral Doppler components; storing the acquired Doppler spectrum data; enabling a user to select a sweep speed; comparing the set repetition period with the selected sweep speed to adjust the Doppler spectrum data; and displaying a Doppler spectrum image based on the adjusted Doppler spectrum data.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound diagnostic system (300) for displaying a Doppler spectrum image, comprising:
a period setting unit (310) operable to set a repetition period;
a Doppler spectrum data acquiring unit (320) operable to compute spectral Doppler components at the set repetition period from the ultrasound data representative of the target object, said computed spectral Doppler components being used to acquire Doppler spectrum data;
a storage unit (330) operable to store the acquired Doppler spectrum data;
a user input unit (340) operable to enable a user to select a sweep speed;
a data adjusting unit (350) operable to compare the set repetition period with the selected sweep speed to adjust the Doppler spectrum data; and
a display unit (360) operable to display a Doppler spectrum image based on the adjusted Doppler spectrum data; **characterized in that** the period setting unit is further operable to calculate a fastest computable time for computing spectral Doppler components from ultrasound data representative of a target object in the ultrasound diagnostic system, and to set the repetition period based on the calculated fastest computable time.

2. The ultrasound diagnostic system of Claim 1, wherein the set repetition period is set by adding a predetermined margin to the fastest computable time.

3. The ultrasound diagnostic system of Claim 2, wherein the data adjusting unit (350) interpolates the Doppler spectrum data when the sweep speed is faster than the set repetition period and decimates the Doppler spectrum data when the sweep speed is slower than the set repetition period.

4. A method of displaying a Doppler spectrum image in an ultrasound diagnostic system, comprising:
calculating a fastest computable time for computing spectral Doppler components from ultrasound data representative of a target object in the ultrasound diagnostic system;
setting a repetition period based on the calculated fastest computable time;
computing the spectral Doppler components at the set period from the ultrasound data representative of the target object;
acquiring Doppler spectrum data based on the computed spectral Doppler components;
storing the acquired Doppler spectrum data;
enabling a user to select a sweep speed;
comparing the set repetition period with the selected sweep speed to adjust the Doppler spectrum data; and
displaying a Doppler spectrum image based on the adjusted Doppler spectrum data.

5. The ultrasound diagnostic method of Claim 4, wherein the set repetition period is set by adding a predetermined margin to the fastest computable time.

6. The ultrasound diagnostic method of Claim 5, wherein, in the step adjusting the Doppler spectrum data, if the selected sweep speed is faster than the set repletion period, then the Doppler spectrum data are interpolated, and if the selected sweep speed is slower than the set repetition period, then the Doppler spectrum data are decimated.

## Patentansprüche

1. Ultraschalldiagnosesystem (300) zur Anzeige eines Doppler-Spektrumbildes, welches Folgendes aufweist:
eine Zeitabschnitts-Einstelleinheit (310), welche in der Lage ist, eine Wiederholdauer einzustellen;
eine Doppler-Spektrumdatenerfassungseinheit (320), welche in der Lage ist, Spektraldopplerkomponenten in der eingestellten Wiederholdauer aus den für das Zielobjekt stellvertretenden Ultraschalldaten zu berechnen, wobei die berechneten Spektraldopplerkomponenten verwendet werden, um Doppler-Spektrumdaten zu erlangen;
eine Speichereinheit (330), welche in der Lage ist, die erlangten Doppler-Spektrumdaten zu speichern;
eine Benutzereingabeeinheit (340), welche in der Lage ist, es einem Benutzer zu ermöglichen, eine Durchlaufgeschwindigkeit auszuwählen;
eine Dateneinstelleinheit (350), welche in der Lage ist, die eingestellte Wiederholdauer mit der ausgewählten Durchlaufgeschwindigkeit zu vergleichen, um die Doppler-Spektrumsdaten einzustellen; und
eine Anzeigeeinheit (360), welche in der Lage ist, ein Doppler-Spektrumbild basierend auf den eingestellten Doppler-Spektrumdaten anzuzeigen;
**dadurch gekennzeichnet, dass**
die Zeitabschnitts-Einstelleinheit des weiteren in der Lage ist, eine schnellste berechenbare Zeit zum Berechnen von Spektraldopplerkomponenten aus den für ein Zielobjekt in dem Ultraschalldiagnosesystem stellvertretenden Ultraschalldaten zu ermitteln und die Wiederholdauer basierend auf der ermittelten schnellsten berechenbaren Zeit einzustellen,

2. Ultraschalldiagnosesystem nach Anspruch 1, wobei die eingestellte Wiederholdauer durch Hinzufügen einer vorbestimmten Differenz bzw. Toleranz zu der schnellsten berechenbaren Zeit eingestellt wird.

3. Ultraschalldiagnosesystem nach Anspruch 2, wobei die Dateneinstelleinheit (350) die Doppier-Spektrumdaten interpoliert, wenn die Durchlaufgeschwindigkeit schneller als die eingestellte Wiederholdauer ist, und die (Doppler-Spektrumdater, dezimiert, wenn die Durchlaufgeschwindigkeit langsamer als die eingestellte Wiederholdauer ist.

4. Verfahren zum Anzeigen eines Doppler-Spektrumbilds in einem Ultraschalldiagnosesystem, welches folgende Schritte aufweist:
Ermitteln einer schnellsten berechenbaren Zeit zum Berechnen von Spektraldoppierkomponeriteri aus für ein Zielobjekt in dem Ultraschalldiagnosesystem repräsentativen Ultraschalldaten;
Einstellen einer Wiederholdauer basierend auf der ermittelten schnellsten berechenbaren Zeit;
Berechnen der Spektraldopplerkomponenten in der eingestellten Dauer aus den für das Zielobjekt repräsentativen Ultraschalldaten; Erlangen von Doppler-Spektrumdaten basierend auf den berechneten Spektraldopplerkomponenten;
Speichern der erlangten Doppler-Spektrumdaten;
Erlaubten eines Benutzers, eine Durchlaufgeschwindigkeit auszuwählen;
Vergleichen der eingestellten Wiederholdauer mit der ausgewählten Durchlaufgeschwindigkeit, um die Doppler-Spektrumdaten einzustellen; und
Anzeigen eines Doppler-Spektrumbildes basierend auf den eingestellen Doppler-Spektrumdaten.

5. Ultraschalldiagnoseverfahren nach Anspruch 4, wobei die eingestellte Wiederholdauer durch Hinzufügen einer vorbestimmten Differenz bzw. Toleranz zu der schnellsten berechenbaren Zeit eingestellt wird.

6. Ultraschalldiagnoseverfahren nach Anspruch 5, wobei in dem die Doppler-Spektrumdaten einstellenden Schritt, wenn die ausgewählte Durchlaufgeschwindigkeit als die eingestellte Wiederholdauer ist, die Doppler-Spektrumdaten interpoliert werden, und wenn die ausgewählte Durchlaufgeschwindigkeit langsamer als die eingestellte Wiederholdauer ist, die Doppler-Spektrumdaten dezimiert werden.

## Revendications

1. Système de diagnostic à ultrasons (300) pour l'affichage d'une image de spectre Doppler comprenant :
une unité de réglage de période (310) agencée pour régler une période de répétition ;
une unité (320) d'acquisition de données de spectre Doppler agencée pour analyser des composantes spectrales Doppler à la période de répétition réglée à partir des données ultrasonores représentatives de l'objet cible, lesdites composantes spectrales Doppler analysées étant utilisées pour acquérir des données de spectre Doppler;
une unité de stockage (330) agencée pour stocker les données de spectre Doppler acquises ;
une unité d'entrée de l'utilisateur (340) agencée pour permettre à un utilisateur de sélectionner une vitesse de balayage ;
une unité d'ajustement de données (350) agencée pour comparer la période de répétition prédéterminée à la vitesse de balayage sélectionnée afin d'ajuster les données de spectre Doppler ; et
une unité d'affichage (360) agencée pour afficher une image de spectre Doppler basée sur les données de spectre Doppler ajustées :
**caractérisé en ce que** l'unité de réglage de période est en outre agencée pour calculer un temps analysable le plus rapide pour analyser des composantes spectrales Doppler issues de données ultrasonores représentatives d'un objet cible dans le système de diagnostic à ultrasons, et pour régler la période de répétition sur la base du temps analysable le plus rapide calculé.

2. Système de diagnostic à ultrasons selon la revendication 1, dans lequel la période de répétition réglée est réglée en ajoutant une marge prédéterminée au temps analysable le plus rapide.

3. Système de diagnostic à ultrasons selon la revendication 2, dans lequel l'unité d'ajustement de données (350) interpole les données de spectre Doppler lorsque la vitesse de balayage est plus rapide que la période de répétition réglée et décime les données de spectre Doppler lorsque la vitesse de balayage est plus lente que la période de répétition réglée,

4. Procédé d'affichage d'une image de spectre Doppler dans un système de diagnostic à ultrasons, comprenant:
le calcul d'un temps analysable le plus rapide pour analyser des composantes spectrales Doppler à partir de données ultrasonores représentatives d'un objet cible dans le système de diagnostic à ultrasons ;
le réglage d'une période de répétition basée sur le temps analysable le plus rapide calculé;
le calcul des composantes spectrales Doppler à la période réglée à partir des données ultrasonores représentatives de l'objet cible;
l'acquisition de données de spectre Doppler basées sur les composantes spectrales Doppler calculées ;
le stockage des données de spectre Doppler acquises ;
la permission à un utilisateur de sélectionner une période de balayage ;
la comparaison de la période de répétition réglée à la vitesse de balayage sélectionnée pour ajuster les données de spectre Doppler, et
l'affichage d'une image de spectre Doppler basée sur les données de spectre Doppler ajustées.

5. Procédé de diagnostic à ultrasons selon la revendication 4, dans lequel la période de répétition réglée est réglée en ajoutant une marge prédéterminée au temps analysable le plus rapide.

6. Procédé de diagnostic à ultrasons selon la revendication 5, dans lequel, dans l'étape d'ajustement des données de spectre Doppler, si la vitesse de balayage sélectionnée est plus rapide que la période de répétition réglée, les données de spectre Doppler sont alors interpolées et, si la vitesse de balayage sélectionnée est plus lente que la période de répétition réglée, les données de spectre Doppler sont alors décimées.
